# EUROPEAN PATENT APPLICATION

(11) **EP 2 556 881 A1**
(43) Date of publication of application: **13.02.2013**
(21) Application number: 11177293.5
(22) Date of filing: 11.08.2011
(51) Int. Cl.: B01D 53/78, B01D 53/62, A01G 33/00, C12N 1/12

(54) **Combining algae cultivation and CO2 capture**

(71) Applicant: Nederlandse Organisatie voor Toegepast -Natuurwetenschappelijk Onderzoek TNO, 2628 VK Delft (NL)
(72) Inventor: Goetheer, Earl Lawrence Vincent, 2628 VK Delft (NL); van den Broeke, Leo Jacques Pierre, 2628 VK Delft (NL); Jahn, Judith, 2628 VK Delft (NL); van den Bos, Willempje Antonie Patricia, 2628 VK Delft (NL); Roelands, Cornelis Petrus Marcus, 2628 VK Delft (NL)
(74) Representative: Jansen, Cornelis Marinus

(57) **Abstract**

The invention relates to the field of culturing algae and use of algae-derived products. The invention also relates to the reduction of CO₂ emission. More in particular the invention relates to combining the capture and conversion of CO₂ from flue gas or biogas and the growth of algae. The invention provides a method for advantageously combining reduction of CO₂ emission with algal growth.

## Description

The invention relates to the field of culturing algae and use of algae-derived products. The invention also relates to the reduction of CO₂ emission and to a composition of a combined absorption liquids and algae growth medium useful for culturing algae.

Commercial and industrial algae cultivation has numerous uses, including production of food ingredients, food, fertilizer, bioplastics, dyes and colorants, chemical feedstock, pharmaceuticals, and algal fuel.

Water, carbon dioxide, minerals and light are all important factors in cultivation, and different algae have different requirements. The basic reaction in water is carbon dioxide + light energy + water = glucose + oxygen + water

The current method for culturing algae is to feed CO₂ to an algae system by bubbling CO₂ through a bioreactor. For an effective contacting of the CO₂ with the liquid phase a considerable amount of energy is required.

There are two main bioreactor types that are being used to cultivate algae, these are open pond reactors and closed bioreactor, the latter are also referred to as photobioreactors. With respect to the photobioreactors, there are two main types and these are tubular reactors and flat panel reactors.

The two most important commercial production processes are using the alga *Chlorella* and the alga *Spirulina* for food supplements. In 2004, China alone produced around 40,000 tons of *Spirulina,* worth about 17 million US dollars.

The costs of nutrients, and especially the carbon nutrients which account for 10 - 25 percent of total operation costs, is a major component of the final production costs of Spirulina biomass.

For commercial production in open race-way ponds some CO2 is obtained from passively absorption. For most commercial production process other kinds of carbon sources (waste, bone meal, etc) are used, instead of contacting with CO2.

One way of cutting down costs on carbon nutrients is to use carbon sources obtained from by-products of for instance coal-fired power plants or bio-degradable waste. For instance, human and animal waste has been used. Although such by-products themselves are generally freely available, storage and transport of waste or other by-products, such as bone-meal, are costly.

Therefore, a continuous source of inorganic carbon, present nearby the algal culture farm is preferred. Such continuous source can for instance be provided by gas from a coal fired power plant or from a biogas plant. The CO₂ production in a coal-fired 500 MW power plant is well over 2.5 million ton of CO₂ per year.

A major advantage of using CO₂ exhaust from a power plant in the culturing of algae, is that less CO₂ is emitted into the atmosphere. As CO₂ is a so called green house gas (GHG) contributing to global warming, use of CO₂ from flue gas in algal culture decreases the amount of GHG and thus contributes to reduction of global warming on the long term.

There are several review papers providing a good overview of the main aspects required for an efficient algae system for biofixation of CO2, including for instance the optimal type of photobioreactor [2], combining CO2 biomitigation and energy production [3], operational aspects of photobioreactors [4], and the production of biofuels from microalgae [5].

The use of algae in bioconversion of CO₂ from flue gas is not new [6, 7, 8, 9, 10]. However, for direct injection of millions of tons of CO₂ per year (which is about 40,000 liter of CO₂ per second) into an algal pond, an open pond in the order of 5-10 km2 of area is required. Therefore, typically, the land mass needed for algal ponds, but also development of supporting systems, like CO₂ injection systems [1], restricts the use flue gas as a carbon source for algal culture. There is thus need for other methods capable of using flue gas or biogas, but requiring much smaller areas of land or smaller bioreactors.

The invention now provides a method for advantageously combining an aqueous absorption liquid for capturing CO₂ with a growth medium normally used to cultivate algae in order to efficiently combine capturing of CO₂, e.g. from flue gas, and bioconversion thereof by algae.

Absorption liquids capable of removing CO₂ from flue gas are known and widely used. On a large scale CO₂ capture is performed with an absorption-stripping process using different types of solvents, including amines and amino acids. The regeneration of the solvent loaded with CO₂ is done by heating the solvent, and this is the most energy consuming step for the full carbon capture and storage (CCS) chain. Thermal regeneration of the solvents accounts for about 75% of the operational cost for the absorption-stripper process. It is estimated that sequestration of CO₂ costs about 50 USD / ton, about three-quarters thereof are needed for thermal regeneration of the solvent, which amounts to approximately 100 million USD for a 500 MW power plant. It is estimated that the costs amount to about 10 - 25% of the total operational costs of a power plant. There is thus clearly a need for a more efficient way of regenerating of solvents.

By combining algae growth with CO₂ capture it is to be expected that CO₂ capture will become more efficient. There will be a clear reduction in the OPEX, because the regeneration of the solvent normally done by heating the solvent well above 100 °C is now performed by the algae at ambient temperature. Furthermore, sulphur and nitrogen containing products, such as SOx and NOx, which are considered environmental pollutants responsible, for instance, for acid rain, can be converted and beneficially used for algal production. The present invention thus provides a method that efficiently combines the reduction of CO₂ emission with growth of algae.

In a first embodiment, the invention provides a method of promoting growth of algae using an absorption liquid comprising carbon dioxide, the method including the steps of:
(1) contacting a gas comprising CO₂ with an absorbent liquid,
(2) allowing the absorbent liquid to absorb carbon dioxide from the gas,
(3) contacting the absorbent liquid comprising the absorbed carbon dioxide to with an algal culture,
(4) allowing the algal culture to convert the carbon dioxide from the absorption liquid, thereby regenerating the absorption liquid and promoting the growth of algae in said algal culture.
   In a method of the invention, it is important that the CO₂ can be "stored" in the absorption liquid solution. The CO₂ is chemically bound and will therefore not be released to the atmosphere as easily when compared to CO₂ dissolved in water. It will thus be possible to have very high CO₂ capture efficiency as compared to, for instance, when CO₂ is bubbled through an aqueous growth medium. One advantage of the use of an absorption liquid is that, e.g. when using an open pond, considerable loss of CO₂ from the open pond is avoided. Second, a method of the invention enables regeneration of the absorption liquid solution without using high amounts of energy, used in conventional methods of regeneration, described above.
   A method according to the invention can be operated both in continuous operation, and batch-wise.
   In a preferred embodiment, a method according to the invention is provided, wherein
   in step (1), a gaseous stream comprising CO₂, preferably flue gas or biogas, is contacted with a liquid stream of absorbent liquid,
   in step (3), the liquid stream is added continuously to the algal culture, the method further comprising the step of:
(5) recycling the regenerated absorbent liquid to be contacted with the gaseous stream in method step (1).

When step 1, 2 and 3 are performed simultaneously, the gaseous stream is added to an aqueous solution comprising the absorption solvent and the algae. In such a method it is for instance possible to continuously add CO₂ to a bioreactor comprising algal and absorption solvent. The absorption solvent prevents loss of CO₂ as it efficiently captures the CO₂ within the algal culture medium. Simultaneously, the algae convert the captured CO₂, thereby regenerating the absorption solvent. When, after a certain period of use, the algal concentration becomes too high, the algae can be harvested and the bioreactor with solvent and (new) algae be reused in the process. In a preferred embodiment, a method according to the invention is provided, wherein steps (1), (2) and (3) are performed simultaneously.

In another preferred embodiment, a method according to the invention is provided, wherein
in step (1), a fixed amount of CO₂ is first added to a batch reactor containing a fixed amount of an absorbent liquid,
in step (2), the fixed amount of absorbent liquid is allowed to absorb the CO₂ for a fixed period of time before in step (3), said absorbent liquid is contacted with the algal culture.

In this configuration, the CO₂ is first stored in an absorption liquid before the solvent comprising the CO₂ is added to an algal culture. One advantage is that for instance, before adding the solvent to the algal culture, quality checks or purification steps can be performed on the solvent. Another advantage is that high concentrations of CO₂ can be stored in relatively small volumes of solvents before the solvent is diluted in the algal culture medium.

In a more preferred embodiment, however, a method according to the invention is provided wherein
a fixed amount of CO₂ is added to a batch reactor containing a fixed amount of absorbent liquid, growth medium and algae, and
the CO₂ is allowed for a fixed period of time to be absorbed by the absorbent liquid and be converted by the algae, before harvesting said algae.

This configuration of batch-wise operation has the advantage that it is easy to reuse the aqueous absorption liquid by first harvesting the algae from the batch reactor and just adding fresh algae and nutrients before adding a new fixed amount of CO₂. In a preferred embodiment, a method according to the invention is provided wherein the regenerated absorption liquid is reused in the method

In a preferred embodiment, a method according to the invention is provided, wherein the carbon dioxide captured in the absorption liquid is from flue gas, preferably comprising between 4 - 25% CO2 and this is amount of CO₂ is balanced by mainly nitrogen (70 - 91%) and some impurities (< 5 %) at atmospheric conditions. In another preferred embodiment, a method according to the invention is provided wherein the carbon dioxide captured in the absorption liquid is from biogas, preferably comprising between 20-50% CO2, balanced by methane (45-75%) and some impurities (< 5%), at a pressure of between 1 and 20 bar.

One way to operate a method according to the invention is to make use of algae that can tolerate high pH, a good example is *Spirulina Platensis.* Absorption liquids with high pH are able to absorb more CO₂ than an absorption liquid with neutral or even acidic pH. This is because, at alkaline pH, e.g. 8.0 and higher, the equilibrium between gaseous CO₂, HCO₃⁻ and CO₃²⁻ is shifted more to the right, enabling more CO₂ to be taken up by the absorption liquid at equal partial CO₂ pressure. In all cases, the absorption liquid can be fresh (no CO₂ present), partially loaded with CO₂, or saturated with CO₂.

In a preferred embodiment, therefore, a method according to the invention is provided, wherein the absorption liquid has a pH of 8.0 or more, preferably of 8.5 or more, more preferably of 9.0 or more, more preferably of 9.5 or more, most preferably of 10.0 or more.

In order for the algae to grow in an alkaline environment, which is beneficial for the absorption of CO₂, the algae must of course be tolerant to such alkaline pH. In a preferred embodiment, therefore, a method according to the invention is provided, wherein said algae tolerate a pH of above 8.0, preferably above 8.5, more preferably above 9.0, more preferably above 9.5, most preferably above 10.0. With tolerate is meant that the algae are at least not irreversibly damaged. Preferably, the algae do not deteriorate in said alkaline absorption liquid. More preferably, the algae are able to thrive in an environment with a pH of above 8.0, preferably above 8.5, more preferably above 9.0, more preferably above 9.5, most preferably above 10.0. Preferred examples of algae which flourish in alkaline pH, i.e. a pH of above 8.0 or higher are *Spirulina platensis, Neochloris oleoabundans, Chlorella vulgaris,* or *Scenedesmus obliquus.* The skilled person is aware of the effect of alkaline pH on other algae species and is able to choose a species that is suitable for use in a method according to the invention.

For some specific applications of the present invention, other characteristics of algae may be very useful. For instance, algae tolerant to other constituents of flue gas, such as NOx and SOx, or of biogas, such as methane, may be very helpful in avoiding the need for pre-removal of these constituents. Also the capability to grow in temperatures well above 40°C may be very useful, as for such algae, no strict temperature control of the absorption liquid, which is generally heated up by the flue gas, is needed before adding the liquid to the algae.

For a good integration of the algae cultivation process and the absorption process on a large scale, different kind of activators for the absorption process can be used.

A carbonate solution, like sodium carbonate can be used to enhance the transfer of CO₂ from the gas phase to the liquid phase [11], an amine, like MEA or an amino acid, like beta-alanine can be used to enhance the reaction rate of CO₂ binding in the amine or amino acid solution. Different activators that maybe used in a method according to the invention are for instance listed in Table II.

For the chemical absorption of CO₂ in a primary amine solution, like mono ethanol amine, MEA (C2H7NO, 2 amino-ethanol) two reactions are relevant:
1) Carbamate formation from CO₂ and MEA (HO-CH₂-CH₂-NH₂),

   CO₂ + 2 HO-CH₂-CH₂-NH₂ = HO-CH₂-CH₂-NH-COO- + HO-CH2-CH2-NH₃⁺
2) Bicarbonate formation for conditions with 6 < pH <11

   CO₂ + HO-CH₂-CH₂-NH₂ + H₂O = HCO₃⁻ + HO-CH₂-CH₂-NH₃⁺

A typical amino acid used for CO₂ capture is beta-alanine (C3H7NO, 2-aminopropanoic acid). Also, for amino acids two reactions are relevant for the binding of CO₂
1) Carbamate formation from CO₂ and beta-alanine (HOOC-CH₂-CH₂-NH₂)

   CO₂ + 2 HOOC-CH₂-CH₂-NH₂ = HOOC-CH₂-CH₂-NH-COO⁻ + HOOC-CH2-CH2-NH3⁺
2) Bicarbonate formation for conditions with 6 < pH <11

   CO₂ + HOOC-CH₂-CH₂-NH₂ + H₂O = HCO₃⁻ + HOOC-CH₂-CH₂-NH₃⁺

In general, it is important to enhance the solubility and the rate of uptake of CO₂ in the solvents. If the solubility and/or the uptake rate (mass transfer and reaction rate) are too low, the absorption column must be enlarged in order to absorb most of the CO2 from the gaseous stream. A column which is too large, is in general too expensive to be economically feasible.

By operating the algae cultivation at high pH, typically above pH 9, the absorption capacity, the amount of CO₂ that can be solubilized, will increase. Not only the solubility of CO₂ in an alkaline medium, like an absorption liquid, is considerably higher than the solubility of CO₂ in water, but also the reaction rate of CO₂ binding to the solvent is considerably higher. For a chemical solvent, like an amine or an amino acid, the solubility of CO₂ is 30 to 40 times higher than the solubility of CO₂ in water. An important aspect is to separate the algae from the absorption liquid to avoid settling of algae in the absorption column.

Besides the cost reduction related to the CO₂ capture process, the combined system, that is absorption liquids integrated with growth medium, can be beneficial for the algae cultivation process, because of a high CO₂ content in the aqueous solution. The integrated system, consisting of the solvent and the growth medium, provides an abundant carbon source.

In one embodiment, therefore, the invention provides a composition comprising a mixture of an algal culture medium and an absorption liquid. Typically a standard algal culture medium is used and mixed 1:1 with an absorption liquid. In a preferred embodiment, the composition comprises about one part standard algal culture medium mixed with one part absorption liquid. In a preferred embodiment, the composition further comprises algae.

The terms "mixture" and "mixed" relate to the addition of the algal culture medium to the absorption liquid or vice versa. It is also possible to mix individual ingredients of the standard growth medium and of the absorption liquid instead of first preparing a growth medium or an absorption liquid. The composition thus refers to the end product, obtainable by mixing an algal culture medium and an absorption liquid, but also by other methods, and is not limited to a composition directly obtained by mixing both the medium and the liquid.

A composition which results in high growth rate, low doubling time, and the high (dry) algae density is for instance obtained with a standard growth medium for a specific algae species and the same amount of an absorption liquid and half of the amount of algae. For a starting solution of 1 L aiming at an initial algae density of 0.2 g/L the following amounts can be used:
- standard growth medium: 400 mL,
- amino acids salts, with as 'counter ion' potassium, also 400 mL, with a given molarity,
- and algae solution: 200 mL with an algae density of about 1 gram/L.

The different growth media used to cultivate algae are well documented [12, 13, 14]. For *Spirulina platensis* different media are used, referred to as 'Medium of Paoletti' or Medium of Schlösser' [13].

Another generally applied medium is the Acidic Bold Basal Medium (ABM). An overview of different recipes for cultivating algae is provided by Culture Collection of Algae and Protozoa (CCAP), and list of about 100 different recipes is available [14]

The specific nutrients used for the growth media are give in Table 2 and Table 3

In a preferred embodiment, a composition according to the invention is provided wherein the composition comprises algal nutrients like Nitrogen (N), Phosphorus (P), and Potassium (K), silica, iron, EDTA and various micro-nutrients. Micro-nutrients include trace elements, trace metals, minerals, and vitamins. For sustainable growth of the micro-algae the N:P ratio should be in the range of 5 to 50, preferably in the range of 10 -40, more preferably in the range of 15 - 35, more preferably in the range of 20 - 30, most preferably about 25.

With respect to the absorption liquid the composition comprises an active component, such as amino acid or amine in a concentration of between 0.1 to 4.0 mol/L (0.1 to 4.0 M), preferably between 0.2 to 3.0 M, more preferably between 0.3 to 2.5 M, more preferably between 0.5 to 2.0 M, most preferably about 1.0 M. As active amino acid component taurine and b-alanine and as active amine component, MEA and DEA have been applied. Other active components used for the absorption liquids are given in Table 4.

There are only a few papers that deal with solvents used in combination with algal growth. All these papers deal with the use of solvents in the recovery, mainly by extraction, of valuable products from the algae [15]. Only two papers [16, 17] describe the effect of solvents on the growth rate of algae. However, the papers only study the effect of traditional organic solvents, including methanol, ethanol, acetone, DMF (dimethyl formamide) and DMSO (dimethyl sulfoxide) and ionic liquids typically used in solvent extraction processes. None of the solvents studied can be used in a large scale absorption process to remove CO₂ from flue gas, because of the limited affinity of these solvent for CO₂.

The present invention for the first time identified solvents capable of both absorbing CO₂ with high affinity and being compatible with use in algal culture.

Algae are categorized into microalgae and macroalgae. Although both algae may be used in the present invention, it is preferred to use microalgae (also referred to as phytoplankton, microphytes, or planktonic algae). Macroalgae, commonly known as seaweed, may also be used, but due to their size and the specific requirements of the environment in which they need to grow, are less preferred.

It is preferred that monocultural algae are used. With mixed cultures, one species may become dominant over time and may change the properties of the algal culture

The water in the algal pond or bioreactor must be in a temperature range that will support the specific algal species being grown. Especially if the pond or bioreactor is to be used throughout the year, it is important to be able to regulate the temperature of the water. In case of stripping CO₂ from flue gas or the like, it is preferred to transfer (at least some of the) heat of the flue gas to the absorption liquid, in order to warm up the pond or bioreactor to an acceptable temperature.

In order to convert CO₂ into oxygen and glucose, algae need light. Direct sunlight is too strong for most algae, which need only about 1/10 the amount of light they receive from direct sunlight. In a dense algal culture, light may only penetrate the top 3 to 4 inches (76-100 mm) of the water. When deeper ponds are used, the water should be agitated, such that the algae are circulated. This prevents both, over-exposure to sun-light and under-exposure, e.g. because algae on the bottom of the pond receive (almost) no light at all. Paddle wheels can stir the water and compressed air coming from the bottom lifts algae from the lower regions. Of course, the continuous stream of the absorption liquid flowing into the pond or bioreactor can also be used to agitate the algae and is much preferred.

Another means of supplying light is to place the light in the system. Glow plates made from sheets of plastic or glass and placed within the tank offer precise control over light intensity.

As already said before, algae can be cultured in open-ponds (such as raceway-type ponds and lakes) and photobioreactors. Raceway-type ponds and lakes are open to the elements and may be contaminated by other microorganisms or chemicals, for instance from nearby plants. Moreover, open ponds are much less controllable with regard to temperature and lighting. Open ponds, however, are cheaper to construct, at the minimum requiring only a trench or pond. Large ponds have the largest production capacities relative to other systems of comparable cost.

One possibility of combining the best of both is for instance to enclose the open pond with a transparent or translucent barrier. This solves many of the problems associated with an open system. One important advantage of such enclosed open pond is that it, when heated, can be used throughout all seasons.

Algae and especially oleaginous algae, such as *Neochloris oleoabundans,* have drawn attention of researchers in the field of biofuel.

Many algae known in the art comprise bio-fuel precursors in the form of lipids or free fatty acids. Preferred species for use in a method according to the invention are *Neochloris oleoabundans* and *Chlorella protothecoides.*

In a preferred embodiment, therefore, a method according to the invention is provided, wherein said algae are oleaginous algae, preferably *Neochloris oleoabunduns* or *Chlorella protothecoides. Neochloris Oleoabundans* is also capable to grow at high pH, which is advantageous for the reasons described above.

In a preferred embodiment, therefore, a method according to the invention is provided, wherein said algal culture comprises *Neochloris oleabundans.*

In another preferred embodiment, a method according to the invention is provided wherein said algal culture comprises *Spirulina platensis, Chlorella vulgaris,* or *Scenedesmus obliquus*

The bio-fuel precursors are then preferably extracted from the algae, whereafter bio-fuel can be produced by conventional methods.

In a preferred embodiment, a method according to the invention is provided, wherein the method further comprises the step of:
- extracting one or more bio-fuel precursors from said algae, preferably followed by the step of:
- preparing bio-fuel from said bio-fuel precursors.

Methods for processing biofuel precursors obtained from algae are known in the art. Conversion of biomass into biofuel product typically encompasses two main routes (thermal and biochemical) [16]. Thermal conversion includes: gasification, liquefaction, pyrolysis, combustion, whereas biochemical conversion includes: digestion, fermentation, and transesterification.

The use of algae is, however, not necessarily restricted to the production of biofuel. Many uses are known in the art. The following examples of algal use should by no means be interpreted as restricting the invention in any way.

Another relevant algae for biofuel production is *Botryococcus Brauni.* This algae is available in different strains, which can be used to produce various long chain hydrocarbons, of the form CₙH_{2n-10,}

The lipids have typically a chain length with n in the range of 30 to 40, and these hydrocarbons can be used in hydrocracking.

Several species of algae are raised for food. Purple laver (Porphyra), for instance is used in nori (Japan), gim (Korea), and laverbread (Wales).

*Spirulina (Arthrospira) platensis* is a blue-green microalgae high in protein and other nutrients and is used as a food supplement. Spirulina can for instance be used as a source of Phycocyanin. Chlorella, is also used as a nutritional supplement with possible effects on metabolic rate. Some allege that Chlorella can reduce mercury levels in humans (supposedly by chelation of the mercury to the cell wall of the organism).

Irish moss (Chondrus crispus), is a source of carrageenan, which can be used as a stiffening agent in instant puddings, sauces, and ice cream, or as a fining agent in beer.

Extracts and oils from algae can also be used as additives in various food products. Most plants produce Omega-3 and Omega-6 fatty acids, which have been shown to have positive health benefits.

Both microalgae and macroalgae can be used to make agar, which is an alternative to animal-derived gelatine.

Other possible uses of algae include the production of bioplastics, dyes, and pharmaceutical ingredients.

In a preferred embodiment, a method according to the invention of promoting growth of algae using an absorption liquid comprising carbon dioxide is provided, wherein the method further comprises the steps of:
- harvesting said algae from said algal culture, and
- extracting one or more chemical species, preferably taken from the group consisting of amino acids, vitamins, such as beta carotene, vitamin B1, B6, B12, E, K1, and K2), minerals, and nutritional and pigment-like compounds , such as poly-saccharides, phycocyanin, and chlorophyll, from said algae.

In a more preferred embodiment, the method further comprises processing of said chemical species. For the processing of 'biochemicals' (dyes, pigments, nutritional species) a wide range of standard methods can be applied, including, mechanical destruction, freezing, membrane filtration, centrifugation, less standard methods are based on ultrasound and supercritical fluid extraction.

The invention is further explained in the following non-limiting examples.

### Figure Legends

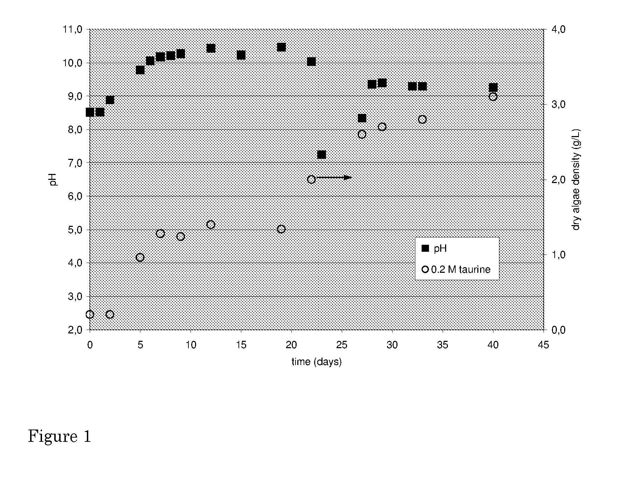

Figure 1. Results for the pH and the dry algae density as function of time, for the alga *Spirulina platensis* is cultivated in a standard solution and using 0.2 M taurine as the absorption liquid.

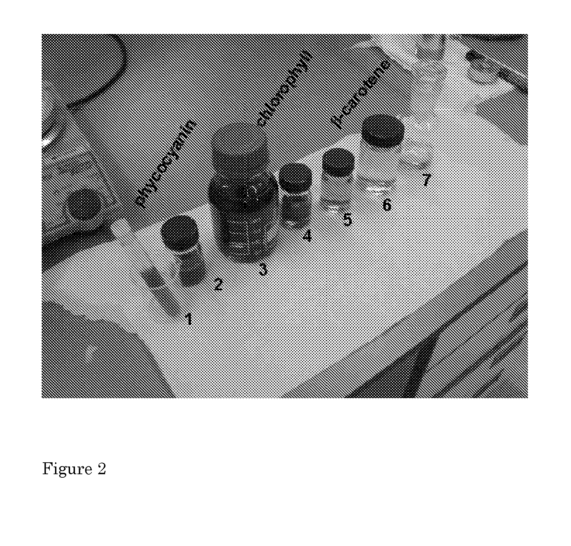

Figure 2. Three main products extracted from *Spirulina platensis* cultivated in a combined nutrients-absorption liquid solution. From left to right the extracted products are phycocyanin (blue solution, sample numbers 1 and 2), chlorophyll (green solution, sample numbers 3, 4 and 5), and ß-carotene (yellow solutions, sample numbers 6 and 7).

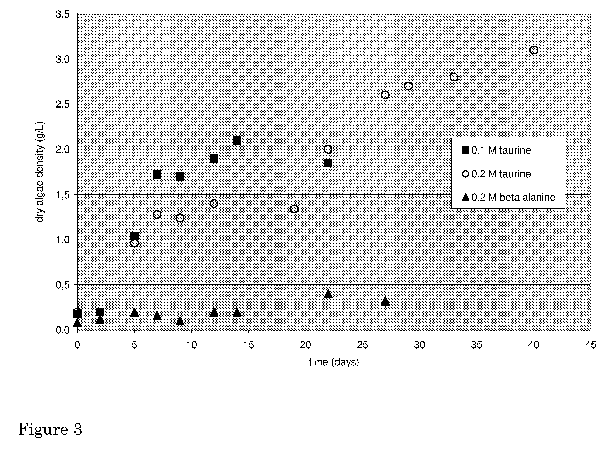

Figure 3. Results for the dry algae density, for Spirulina Platensis, for two different solvents and for different molarity. For the situation with 0.1 M taurine the dry algae density increases to about 2 g/L. For the situation of 0.2 an initial level of dry alga density of about 1.4 is obtained, at day 20 the solution (alga, nutrients, absorption liquid) is loaded again with CO₂, and eventually a dry alga density of just above 3.0 is obtained.

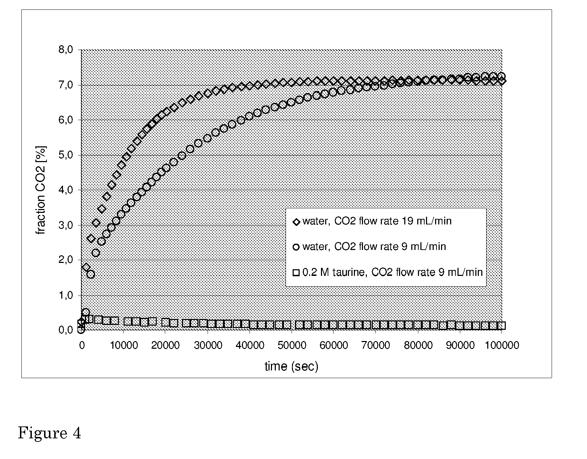

Figure 4. Results for the uptake of CO₂ in two different solutions. CO2 is fed continuously to a sealed beaker with water and with an absorption liquid (0.2 m taurine) The height of the liquids is 30 cm the total amount of liquids is 4L. For water the uptake, that is the amount of CO₂, in %, leaving the beaker as function of the time, is given for two flow rates. For the highest flow rate, 19 mL of CO₂ per minute equilibrium is reached after about 40 000 seconds, while for the lowest flow rate, 9 mL of CO₂ per minute equilibrium is reached after 80 000 seconds. For the third case, the case with the absorption liquid almost no CO₂ is leaving the beaker, and this holds for the total period of time (100 000 seconds, almost 28 hours).

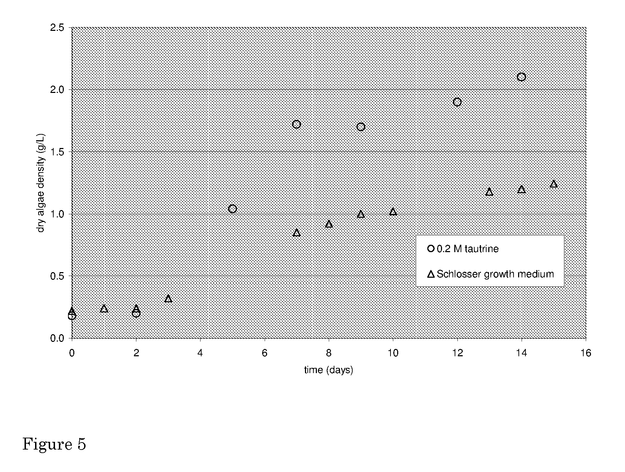

Figure 5. Results for dry algae density as a function of time for two cases. A comparison is made between growth obtained in the presence of 0.2 M taurine and CO2, and growth obtained for standard conditions, aqueous solution, white light and no CO2 added.

### Examples

### Materials and Methods

To cultivate the various micro-algae growth media recipes given in the open literature have been used. A number of typical recipes for growth media are given in Table 2 and Table 3.

For the absorption liquids standard procedures were used to prepare the solvent solutions. For the absorption liquids stock solutions with a molarity of 2 M were prepared. To test the growth, the molarities of the solutions were adjusted at the start of the experiments by adding a given amount of the aqueous growth medium.

The growth of the algae was tested with different solutions composed from different ratios (volume based) of growth media and absorption liquid. This resulted in values for the molarity for the absorption liquid, in the combined solution, of 0.1 M, 0.2 M, 0.3 M, 0.4 M, 0.5 M, 0.6 M, 0.7, M, 0.8 M, 0.9 M, and 1.0 M.

Initially, so-called toxicity tests were performed to determine to what value of the molarity of the absorption liquids algae growth was possible. It is noted that these experiments were performed without adding CO₂ to the solution.

Subsequently, a number of dedicated experiments were performed were a specific algae (and growth medium) was combined with a given absorption liquid in the presence of CO₂. The CO₂ was added in two different ways.

In the first way, so-called batch operation, the algae, growth medium, and the absorption liquid (with a given molarity) were prepared in a beaker (of about 1 L) or reactor (available with a volume of 10 L and 20 L) and the CO₂ was bubbled through for at least 12 hours to saturate the solution with a high flow rate (in the order of 200 mL/min).

In the second, way the same solution was used, composed of the micro-algae, growth medium, absorption liquid, and the CO₂ was bubbled through the solution in the beaker (or reactor) continuously at a given flow rate (in the order of 10 to 20 mL/min).

During the experiments the pH was measured on a daily basis.

At given times a small sample was taken from the beaker (or reactor) to analyse the micro-algae. The volume of the sample was determined, the sample was then dried in an oven, and subsequently the mass determined (by weighing) to obtain the dry algae density (g/L).

### Example I

In Figure 1 the pH and the dry algae density as function of the time is depicted. Results are for *Spirulina platensis* and using the amino acid taurine, 0.2 M, as the active component for the absorption liquid.

At the start of the experiment the aqueous solution with the nutrients, the micro-alga, and the taurine is first saturated with CO₂ by bubbling CO₂ through the solution for at least 12 hours. The alga starts to grow after 3 to 4 days, reaching a constant level at a density of about 1.4 g/L (dry alga mass). At day 20 the solution (alga, nutrients, taurine) is loaded again with CO₂. The CO₂ is bubbled through the solution overnight for at least 12 hours. After day 20, the alga starts to grow again to reach a final density of about 3.0 g/L (dry alga density).

For most of the time, the pH of solution is above 8.0, and only between day 20 and day 25 the pH drops to a value below 8.0, but after day 25 the pH starts to increases again to reach a final value for the pH of just above 9.0.

### Example II

Extraction of different species, including β-carotene, chlorophyll, and phycocyanin, from *Spirulina platensis.*

An example of a relevant biochemical that can be extracted from *Spirulina platensis* is phycocyanin. Phycocyanin is being used for different applications in the pharmaceutical, food, and beverage industry. Phycocyanin is used in the food and beverage industry as a natural coloring agent.

### Example III

A number of solvents have been combined with well-know recipes for growth media for different algae to see if is possible to grow micro-algae in the new solution combining growth media and an absorption liquid. The composition of the solution was composed in such a way that the molarity of the absorption liquid was in the range of 0.1 to 1.0 M.

In Figure 3 three different curves are shown for the density of the dry density of the *Spirulina Platensis.*

Results have been obtained for two different solvents and for different molarity. For the situation with 0.1 M taurine the dry algae density increases to about 2 g/L. For the situation of 0.2 an initial level of dry alga density of about 1.4 is obtained, at day 20 the solution (alga, nutrients, absorption liquid) is loaded again with CO2, and eventually a dry alga density of just above 3.0 is obtained. For the third curve, obtained using 0.2 M taurine, no algae growth was observed.

This clearly indicated that the solvent can be reloaded with CO₂ and that the solvent can be re-used.

### Example IV (Composition growth medium + absorption liquid)

For various tests, *Arthrospira (Spirulina) Platensis* is used. The spirulina strain is kept in Schlössers medium [Schlösser, U.G., Ber. Deut. Bot. Ges. 95 (1982) 181-276]. Schlössers medium contained per liter: 13.6 g NaHC03, 4.03 g Na2CO3, 0.5 g K2HP04, 2.5 g NaN03, 1 g K2SO4, 1 g NaCl, 0.2 g MgS04.7aq, 0.04 g CaC12.2aq, 0.01 g FeS04.7aq, 0.08 g EDTA. Some micronutrients are added as well.

The overall composition based on 1 L of total solution is obtained by combining: 400 mL of the growth medium, for example the Schlössers medium, with 400 mL of an aqueous amino acids salts, with as 'counter ion' potassium hydroxide, solution, where the amount of amino acid is varied between 0.1 and 1 M, subsequently, 200 mL of algae solution is added.

There are several different test set-ups, glass reactors, to cultivate spirulina. All set-ups consists of stirring equipment, single or double walled beaker, fermentor, or aquaria, with external heating in case of the double walled aquaria and light sources. A flexible gas supply system, to add the CO2, to the different set-ups is installed.

With respect to the size of the glass reactors, reactors are available that can contain a solution with a volume of approximately 1 L, 6 L, 12 L, 20 L, and 35 L.

The temperature of the algal cultures is set to vary between 25 to 35 °C.

### Example V (CO2 uptake in water)

In Figure 4 three curves are depicted to show the influence of the CO₂ flow rate and the effect of the presence of an absorption liquid, in this case, taurine, on the uptake of CO₂ in the aqueous solution. In Figure 4 the CO₂ concentration, in %, leaving the beaker as function of time is depicted.

Experiments have conducted in a sealed beaker, where flue gas was fed, and where the gaseous stream leaving the beaker was analyzed with a on-line CO₂ analyzer. The composition of the 'flue gas' was 7.2 % carbon dioxide CO2 and 92.3 % nitrogen (N2). In the case of water there is a clear CO₂ concentration measured, after about 10 000 seconds, for both cases of water, the fraction is above 3.5 %. This indicates that at least 50 % of the CO₂ fed to the beaker is leaving the beaker. In the case of the absorption liquid no CO2 is measured, indicating that all CO2 remains absorbed in the liquid.

It is clear that the absorption liquid can be used to store the CO₂ over considerable time range.

### Example VI (Comparison growth rate with and with out absorption liquid present)

In Figure 5 a comparison is made between the dry algae density of Spirulina obtained for the case with the absorption liquid, 0.2 M taurine, initially saturated with CO₂ and the density for the situation that the standard growth medium is used (see also Example IV).

Initially, there is some start-up effect of about two to three days for both cases, and after this period the density increase in the presence of the absorption liquid from 0.25 to 1, 7 in about 5 days, while for the other situation the density increases from 0.25 to about 1 in 9 days. This gives a doubling time

There is also a clear effect in the finally density, after about 15 days, that is 2.1 g/L versus 1.4 g/L.

**Table I List of the four micro-algae tested in combination with absorption liquids**

| Micro-algae | |
|---|---|
| | Spirulina platensis |
| | Neochloris oleoabunduns |
| | Chlorella vulgaris |
| | Scenedesmus obliquus |

**Table II List of nutrients used for the cultivation of Spirulina Platensis. Two different recipes are described in the literature [14]**

| Species | Schlösser (g per L) | Paoletti (g per L) |
|---|---|---|
| NaHC03 | 13.6 | 15.2 g |
| Na2CO3 | 4.03 | 8.89 g |
| K2HP04 | 0.50 | 0.50 |
| NaN03 | 2.50 | |
| K2SO4, | 1.00 | |
| NaCl, | 1.00 | 0.92 g |
| MgS04.7aq, | 0.20 | 0.25 g |
| CaC12.2aq, | 0.04 | 0.05 g |
| KN03 | | 2.57 |
| Na2SO4 | | 1.88 |
| EDTA solution | see below | |
| EDTA solution | | see below |
| Micronutrient solution | P-IV (see below) | |
| Micronutrient solution | CHU (see below) | |
| Micronutrient solution | | see below |

For the Schlösser medium, EDTA is added as part of the micronutrient solution. Also, for the Schlösser medium prepare, 6.0 mL of P-IV metal solution, with the following species in (g/L)

| | |
|---|---|
| 0.75 | Na2EDTA.2aq |
| 0.097 | FEC13.6aq |
| 0.041 | MnC12.4aq |
| 0.005 | ZnCl2 |
| 0.002 | CoCl2.6aq |
| 0.004 | Na2MoO4.2aq |

Subsequently prepare, for the Schlösser medium, 1.0 mL of CHU micronutrient solution with the following species in (g/L)

| | |
|---|---|
| 0.020 | CuSO4.5aq |
| 0.044 | ZnS04.7aq |
| 0.020 | CoCl2.6aq |
| 0.012 | MnCl2.4aq |
| 0.012 | Na2MoO4.2aq |
| 0.620 | H3BO3 |
| 0.050 | NA2EDTA.2aq |

For the Paoletti medium prepare, 1.0 mL of Fe-EDTA solution, with the following species in (g/L)

| | |
|---|---|
| 29.8 | EDTA-Na2 |
| 24.9 | FeS04.7aq |

Subsequently prepare, also for the Paoletti medium, 1.0 mL of micronutrient solution with the following species in (g/L)

| | |
|---|---|
| 2.85 | BaCl2.2aq |
| 0.19 | CoCl2.6aq |
| 0.17 | CoCl2.6aq |
| 0.14 | SeCl2.2aq |
| 0.30 | SnCl2.2aq |
| 0.29 | LiCl |
| 0.18 | CuSo4.5aq |
| 0.71 | NiSo4.5aq |
| 0.12 | NaMoO4.2aq |

**Table III List of nutrients for the ABM (Acidic Bold Basal Medium with Vitamins; modified) medium. The recipe is described in [20].**

| To prepare the medium, dissolve 250 mg (NH4)2SO4 in 800 ml distilled water and add the following using a stock solutions in g / 1000 mL water for 1 L final medium | | |
|---|---|---|
| Species | Amount | Volume |
| NaN03 | 25.0 g | 30.0 mL |
| CaC12.2H2O | 2.5 g | 10.0 mL |
| MgS04.7H2O | 7.5 g | 10.0 mL |
| K2HP04.3H2O | 7.5 g | 10.0 mL |
| KH2PO4 | 17.5 g | 10.0 mL |
| NaCl | 2.5 g | 10.0 mL |
| trace element solution (see below) | | 6.0 mL |
| vitamin B1 (see below) | | 1.0 mL |
| vitamin B12 (see below) | | 1.0 mL |

Prepare 1 L with distilled water and adjust the pH to 3.0 with HCl.

### Trace element solution

Add to 1000 mL of distilled water 0.75 g Na2EDTA and the minerals:

| | | |
|---|---|---|
| 97.0 mg | FeC13.6H2O | |
| 41.0 mg | MnC12.4H2O | |
| 5.0 mg | | ZnC12.6H2O |
| 2.0 mg | | CoCl2.6H2O |
| 4.0 mg | | Na2MoO4.2H2O |

### Vitamin B1

0.12 g Thiaminhydrochloride in 100 mL distilled water. Filter sterile.

### Vitamin B12

0.1 g Cyanocobalamin in 100 mL distilled water, take 1 mL of this solution and add 99 mL distilled water. Filter sterile.

**Table IV List of 10 absorption liquids used to test the growth of different micro-algae. The absorption liquids have been used with a molarity in the range of 0.1 M to 1.0 M. Growth of the algae has been tested with and with out the absorption liquids loaded with CO₂**

| Solvents | |
|---|---|
| Amino acids | beta-alanine |
| | sarcosine |
| | 6 amino hexanoic acid |
| | taurine |
| | L glutamic acid |
| Amines | mono ethanol amine (MEA) |
| | di ethanol amine (DEA) |
| | methyl di-ethanol amine (MDEA) |
| Carbonates | K₂CO₃ |
| | Na₂CO₃ |
| | NH₄HCO₃ |

### References

[1] J Benemann, PM Pedroni, J Davison, H Becket, P Bergman, Technology roadmap for biofixation of CO2 and greenhouse gas abatement with microalgae. Proceedings Second Annual Conference on Carbon Sequestration, paper # 017 (www.netl.doe.gov/publications/proceedings/03/carbon-seq/PDFDs/Mainmenu.pdf).
[2] CU Ugwu, H Aoyagi, H Uchiyama, Photobioreactors for mass cultivation of algae. Bioresources Technology 99 (2008) 4021.
[3] B Wang, CO2 biomitigation using microalgae, Applied Microbiology Biotechnology, 79 (2008) 707.
[4] E Jacob-Lopes, S Revah, S Hernandez, K. Shirai, TT Franco, Development of operational strategies to remove carbon dioxide in photobioreactors. Chemical Engineering Journal, 153 (2009) 120.
[5] L Brennan, P Owende, Biofuels from microalgae- A review of technologies for production, processing, and extraction of biofuels and coproducts. Renewable and Sustainable Energy Review 14 (2010) 557
[6] M Feng, Microalgae cultivation in bioreactors for CO2 mitigation from power plant flue gas and fuel production by supercritical CO2 fixation. AIChE 2008 Spring Meeting, Extended Abstract.
[7] E Ono and JL Cuello, Selection of optimal microalgae for CO2 sequestration. Proceedings Second Annual Conference on Carbon Sequestration, paper # 158 (www.netl.doe.gov/publications/proceedings/03/carbon-seq/PDFDs/Mainmenu.pdf).
[8] J Doucha, F Straka, K Livansky, Utilization of flue gas for cultivation of microalgae (Chlorella Sp) in an outdoor open thin-layer photobioreactor. Journal of Applied Phyocology, 17 (2005) 403.
[9] C Stewart M-A Hessami, A study of methods of carbon dioxide capture and sequestration- the sustainability of a photosynthetic bioreactor approach. Energy Conversion and Management 46 (2005) 403.
[10] MG de Morais, JAV Costa, Isolation and selection of microalgae from coal fired thermoelectric power plant for biofixation of carbon dioxide. Energy Conversion and Management 48 (2007) 2169.
[11] HT Hsueh, H Chu, St Yu, A batch study on the bio-fixation of carbon dioxide in the absorbed solution from a chemical wet scrubber by hot spring and marine algae. Chemosphere 66 (2007) 878.
[12] T Mutanda, D Ramesh, S Karthikeyan, S Kumari, AN Anandraj, F. Bux, Bioprospecting for hyper-lipid producing microalgal strains for sustainable biofuel production. Bioresources Technology 102 (2011) 57.
[13] MS Rodrigues, LS Ferreira, A Converti, S Sato, JCM de Carvalho, Influence of ammonium sulphate feedinf time on fed -batch Arthrospira (Spirulina) platensis cultivation and biomass composition with and without PH control. Bioresources Technology 102 (2011) 6587
[14] www.ccap.ac.uk/media/pdfrecipes.htm
[15] F-Y Lee, C Yoo, S-Y Jun, C-Y Ahn, H-M Oh, Comparison of several methods for effective lipid extraction form microalgae. Bioresources Technology 101 (2010) 575.
[16] Y Okumura, J Koyama H Takaku, H Satoh, Influence of organic solvents on growth of marine microalgae. Archives of Environmental Contamination and Toxicology. 41 (2001) 123.
[17] C-W Cho, Y-C Jeon, TPT Pham, K Vijayaraghavan, Y-S Yun, The ecotoxicity of ionic liquid and traditional organic solvents on microalgae Selenastrum capricornutum. Ecotoxicology and Environmental Safety 71 (2008) 166.
[18] NH Tran, JR Bartlett, GSK Kannangara, AS Milev, H Volk, MA Wilson, Catalytic upgrading of biorefinery oil from micro-algae. Fuel, 89 (2010) 265.
[19] L Brennan, P Owende, Biofuels from microalgae - A review of technologies for production, processing, and extractions of biofuels and coproducts, Renewable and Sustainable Energy Reviews 14 (2010), 557.
[20] A Pollio P Cennamo, C Ciniglia, M de Stefano, G Pinto, VAR Huss. Chlamydomonas pitschmannii Ettl, a little known species from thermoacidic Environments. Protist 156 (2005) 287.

## Claims

1. A method of promoting growth of algae using an absorption liquid comprising carbon dioxide, the method including the steps of:
(1) contacting a gas comprising CO₂ with an absorbent liquid,
(2) allowing the absorbent liquid to absorb carbon dioxide from the gas,
(3) contacting the absorbent liquid comprising the absorbed carbon dioxide to with an algal culture,
(4) allowing the algal culture to convert the carbon dioxide from the absorption liquid, thereby regenerating the absorption liquid and promoting the growth of algae in said algal culture.

2. A method according to claim 1, wherein
in step (1), a gaseous stream comprising CO₂, preferably flue gas or biogas, is contacted with a liquid stream of absorbent liquid,
in step (3), the liquid stream is added continuously to the algal culture, the method further comprising the step of:
(5) recycling the regenerated absorbent liquid to be contacted with the gaseous stream in method step (1).

3. A method according to claim 1 or 2, wherein steps (1), (2) and (3) are performed simultaneously.

4. A method according to claim 1, wherein
in step (1), a fixed amount of CO₂ is first added to a batch reactor containing a fixed amount of an absorbent liquid,
in step (2), the fixed amount of absorbent liquid is allowed to absorb the CO₂ for a fixed period of time before in step (3), said absorbent liquid is contacted with the algal culture.

5. A method according to claim 3, wherein
a fixed amount of CO₂ is added to a batch reactor containing a fixed amount of absorbent liquid, growth medium and algae, and the CO₂ is allowed for a fixed period of time to be absorbed by the absorbent liquid and be converted by the algae, before harvesting said algae.

6. A method according to any one of claim 1-5, wherein
- the carbon dioxide captured in the absorption liquid is carbon dioxide from flue gas, preferably comprising 5 - 25% CO₂, balanced mainly by nitrogen (70 - 90%) at atmospheric conditions, or wherein
- the carbon dioxide captured in the absorption liquid is carbon dioxide from biogas, preferably comprising 20-50% CO₂, balanced by mainly methane (45-75 %) at a pressure of between 1 - 20 bar.

7. A method according to any one of claims 1-6, wherein said absorption liquid is first added to an aqueous solution used for culturing algae, before said absorption liquid is added to the algal culture.

8. A method according to any one of claims 1-7, wherein said algae tolerate a pH of above 8.0.

9. A method according to any one of claims 1-8, wherein said absorption liquid has a pH of above 8.0.

10. A method according to claim 8 or 9, wherein said aqueous solution, used for culturing algae, has a pH of above 8.0.

11. A method according to any one of claims 1-10, wherein said algal culture comprises *Spirulina platensis, Neochloris oleoabunduns, Chlorella vulgaris,* or *Scenedesmus obliquus.*

12. A method according to any one of claims 1-11, the method further comprising the steps of:
- harvesting said algae from said algal culture, and
- extracting one or more biofuel precursors and/or one or more chemical species, preferably taken from the group consisting of amino acids, vitamins, such as beta carotene, vitamin B1, B6, B12, E, K1, and K2), minerals, and nutritional and pigment-like compounds , such as poly-saccharides, phycocyanin, and chlorophyll, from said algae, preferably the method further comprising:
- preparing biofuel from said biofuel precursors.

13. A method according to any one of claims 1 - 12, wherein said absorption liquid comprises an amino acid, preferably selected from the group consisting of beta-alanine, sarcosine, 6-amino-hexanoic acid, taurine and L-glutamic acid, an amine, preferably selected from the group consisting of mono-etahanol amine (MEA) and methyldi-ethanol amine (MDEA), or carbonates, preferably selected from the group consisting of K₂CO₃, Na₂CO₃, and NH₄HCO₃.

14. A composition comprising a mixture of an algal culture medium and an absorption liquid, preferably wherein the composition comprises about one part standard algal culture medium mixed with one part absorption liquid.

15. A composition according to claim 14, wherein the composition comprises algal nutrients like Nitrogen (N), Phosphorus (P), and Potassium (K), silica, iron, EDTA, and micro-nutrients like trace elements, trace metals, minerals, and vitamins.
